# FASCICULE DE BREVET EUROPEEN

(11) **EP 3 744 387 B1**
(45) Date de publication et mention de la délivrance du brevet: **23.08.2023**
(21) Numéro de dépôt: 20176951.0
(22) Date de dépôt: 27.05.2020
(51) Int. Cl.: A61N 1/36, A61H 1/02, B62K 3/16, A61N 1/05, A63B 21/00, A63B 22/06, A63B 23/035, A61N 1/04

(54) **VÉHICULE À PÉDALES COMPORTANT UN DISPOSITIF DE STIMULATION ÉLECTRIQUE**
TRETFAHRZEUG (MIT PEDALEN), DAS EINE VORRICHTUNG ZUR ELEKTRISCHEN STIMULATION UMFASST
VEHICLE WITH PEDALS COMPRISING A DEVICE FOR ELECTRICAL STIMULATION

(30) Priorité: 27.05.2019 CH 6862019
(43) Date de publication de la demande: 02.12.2020
(73) Titulaire: GBY SA, 1696 Vuisternens-en-Ogoz (CH)
(72) Inventeur: Tobler, Sebastian, 1726 Farvagny-le-Petit (CH)
(74) Mandataire: P&TS SA (AG, Ltd.)

(56) Documents cités:
- WO-A1-02/28700
- WO-A1-2019/066426
- US-A- 4 499 900
- US-A- 4 863 157

## Description

### Domaine technique

La présente invention concerne un véhicule à pédales adapté pour des personnes limitées dans leur mobilité. Le véhicule à pédales peut par exemple être utilisé par des personnes ayant un ou plusieurs membres paralysés, ou tout au moins partiellement paralysés, par des personnes amputées et portant une prothèse ou par des personnes diminuées physiquement, notamment par la sclérose en plaque ou par la maladie de Lyme.

### Etat de la technique

Il est déjà connu d'utiliser la stimulation électrique de muscles ou de nerfs dans des systèmes de rééducation afin d'améliorer la capacité motrice et la masse musculaire des membres invalides ou partiellement invalides d'une personne ayant subie une liaison au moins partielle de la moelle épinière. L'accroissement de la masse musculaire des membres invalides ou partiellement invalides permet de diminuer le risque de complications, par exemple le risque d'escarre.

KR20140128487 et KR20170098058 divulguent par exemple des appareils de rééducation dans lesquels des électrodes en contact avec les membres d'un utilisateur permettent de mesurer la force exercée par ces membres sur un appareil de rééducation. D'autres électrodes sont prévues pour stimuler électriquement les muscles de membres paralysés.

WO14136852 divulgue un appareil de stimulation électrique permettant la synchronisation d'exercice entre un membre sain et un membre non valide d'un patient et l'application d'une stimulation électrique sur le membre invalide sur la base du mouvement du membre sain.

US2004259693 divulgue un vélo muni de manivelles pour les bras et de pédales pour les pieds permettant la stimulation électrique d'un membre de l'utilisateur.

US9114255 divulgue un dispositif comprenant un corset pouvant être utilisé conjointement avec une stimulation électrique neuromusculaire pour induire des contractions isométriques dans un ou plusieurs muscles des muscles des membres d'un sujet par le biais d'une série de cycles de stimulation. Le dispositif peut comprendre un contrôleur pour stimuler les muscles de la jambe et pour régler de manière autonome la stimulation sur la base d'une force mesurée exercée par la jambe sur le corset. Le contrôleur peut ajuster la stimulation en fonction de la force mesurée d'un cycle précédent par rapport à une force cible.

US4863157 divulgue un tricycle comprenant un guidon avec des poignées entrainées en rotation par les mains. Une chaîne relie mécaniquement l'axe de ces poignées avec la roue avant du tricycle qui est ainsi mise en rotation avec les poignées. Par ailleurs, un capteur détecte la position des poignées et génère un signal afin de stimuler électriquement les muscles des jambes qui sont ainsi actionnées de manière synchronisée avec les bras pour actionner des pédales afin de faire avancer le tricycle également avec les jambes. La stimulation des jambes dépend uniquement du mouvement des bras ; cette stimulation peut être excessive et résulter en une fatigue rapide, notamment lorsque les jambes sont encore peu musclées ou que le véhicule se déplace à la montée. A la descente, les jambes sont stimulées même s'il n'est pas nécessaire d'accélérer le véhicule.

Une solution similaire et qui présente les mêmes inconvénients est décrite dans WO-A1-02/28700.

US8923978 divulgue un appareil d'exercice de rééducation comprenant des éléments de stimulation électrique pouvant être appliqués à un muscle associé au mouvement d'au moins une jambe alors qu'on mesure la force générée par le mouvement d'au moins un bras de la personne de manière à ajuster la stimulation appliquée en fonction de la force mesurée, de manière à obtenir un pourcentage prédéterminé entre la force fournie par les membres supérieurs et les membres inférieurs stimulés.

La régulation tient compte uniquement de la force appliquée par les bras et les jambes. Lorsque l'utilisateur actionne ses bras rapidement, les jambes tournent aussi à vitesse élevée ce qui peut résulter en une fatigue rapide même si la force est faible.

D'autre part, la stimulation électrique des jambes dans le dispositif de US8923978 ne tient pas compte de la participation musculaire volontaire du muscle stimulé. Il arrive cependant dans de nombreux handicaps, en particulier dans le cas de paraplégie partielle, qu'un membre dit invalide soit néanmoins capable de développer une certaine puissance de manière intentionnelle, c'est-à-dire en l'absence de toute stimulation. Le dispositif de US8923978 ne permet pas de tenir compte de cette puissance développée intentionnellement et n'indique pas comment les muscles doivent être stimulés lorsqu'ils développent une puissance intentionnelle non négligeable.

Un autre désavantage de US8923978 provient du fait qu'il s'agit d'un home trainer destiné à la rééducation de personnes dans une salle. La rééducation peut devenir à la longue répétitive et lassante, ce qui peut influencer la motivation de personnes en rééducation. Or, un manque de motivation a un impact négatif important sur l'amélioration des capacités motrices de membres invalides ou partiellement invalides.

Un but de la présente invention est par conséquent de proposer un système de stimulation électrique, pour la rééducation de personnes limitées dans leur mobilité, qui a l'avantage d'être récréatif afin maintenir la motivation des personnes tout au long de leur programme de rééducation.

Un autre but de l'invention est de proposer un système de rééducation avec un système de régulation de la stimulation électrique amélioré.

### Bref résumé de l'invention

Ces buts de l'invention sont atteints gracê à un véhicule à pédales selon la revendication 1. Dans la suite de cette description, toutes les méthodes de stimulation décrites ne font pas partie de l'invention mais servent uniquement des propos illustratives.

On décrit ici une méthode de stimulation électrique d'un ou plusieurs membres d'un utilisateur d'un véhicule à pédales destiné à se déplacer le long d'un parcours au gré des envies de l'utilisateur, le véhicule à pédales comportant un premier organe d'entrainement adapté pour être actionné par les membres supérieurs de l'utilisateur, un second organe d'entrainement adapté pour être actionné par les membres inférieurs de l'utilisateur, ainsi qu'un dispositif de stimulation électrique comprenant un module de commande et au moins une électrode adaptée pour stimuler au moins un membre inférieur destiné à être stimulé, le module de commande étant configuré pour traiter des données relatives à l'intention de l'utilisateur, la méthode consistant à transmettre à ladite au moins une électrode par le module de commande des stimulations électriques en fonction des données relatives à l'intention de l'utilisateur,
dans lequel lesdites données relatives à l'intention de l'utilisateur comprennent la puissance ou la force développée intentionnellement par les membres supérieurs de l'utilisateur,
dans lequel la méthode comporte une étape de détermination de la force ou de la puissance développée intentionnellement par les membres inférieurs,
et en ce que lesdites stimulations électriques sont déterminées de manière à assurer une relation prédéterminée entre la puissance développée par les membres supérieurs et la puissance totale développée par les membres inférieurs, ladite puissance totale comprenant la puissance non nulle développée intentionnellement par les membres inférieurs et la puissance développée par les membres inférieurs grâce à la stimulation.

Selon un premier aspect, la méthode permet ainsi de contrôler la puissance développée par les membres inférieurs en fonction de la puissance développée par les membres supérieurs valides. La fatigue musculaire dépend avant tout de la puissance, qui est proportionnelle à la force uniquement dans l'hypothèse d'une vitesse de rotation constante. Cette hypothèse d'une vitesse de rotation constante est approximativement valide dans le cas d'un tricycle ou d'un engin stationnaire sur lequel l'utilisateur tend à s'entraîner à vitesse constante, mais ne l'est pas dans le cas d'un véhicule tout-terrain amené à être employé au plat, en descente ou à la montée. Le rapport entre la puissance et la force varie aussi dans le cas d'un véhicule comprenant des vitesses, par exemple un dérailleur, permettant de varier le rapport couple-vitesse de rotation pour une puissance donnée.

Selon un autre aspect, la stimulation appliquée aux membres inférieurs tient en outre compte de la puissance développée intentionnellement (c'est-à-dire en l'absence de stimulation) par ces membres inférieurs, notamment dans le cas d'une paraplégie partielle. On évite ainsi de surstimuler les membres inférieurs lorsque ceux-ci peuvent exercer une puissance non nulle même en l'absence stimulation.

Les stimulations électriques peuvent ainsi être déterminées en tenant compte à la fois de la puissance respectivement de la force développée intentionnellement par les membres supérieurs de l'utilisateur et de la puissance respectivement de la force développée intentionnellement par les membres inférieurs de l'utilisateur.

Une détermination de la force développée par les membres supérieurs et par les membres inférieurs peut être appliquée notamment lorsque les organes d'entraînement supérieurs et inférieurs sont couplés mécaniquement. Dans ce cas, une simple mesure de force permet de garantir une proportionnalité entre la puissance exercée par les membres supérieurs et les membres inférieurs, puisque les vitesses de rotation sont liées entre elles.

Une détermination de la puissance développée par les membres supérieurs et par les membres inférieurs est préférable notamment lorsque les vitesses de rotation des organes d'entraînement inférieurs et supérieurs ne sont pas liées entre elles par un facteur connu. Dans ce cas, une détermination de la puissance développée par les membres supérieurs et les membres inférieurs permet une régulation des signaux de stimulation qui tient compte non seulement des forces exercées, mais aussi des vitesses de rotation, afin par exemple de réduire le risque de fatigue ou de blessure en cas de puissance exercée trop importante.

La mesure de puissance sur un organe d'entraînement peut mettre en oeuvre un capteur de couple ou de force et de capteur de vitesse de rotation.

Les stimulations électriques peuvent être déterminées de manière à assurer une proportion prédéterminée entre la force ou la puissance développée intentionnellement par les membres supérieurs de l'utilisateur et la force ou la puissance totale développée par les membres inférieurs.

Les stimulations électriques sont avantageusement stoppées ou réduites lorsque la puissance totale développée par un membre inférieur excède une valeur prédéterminée.

Les stimulations électriques sont avantageusement stoppées ou réduites dès que la mesure de la vitesse de rotation de l'un des premier et second organes d'entrainement excède une vitesse de rotation prédéterminée. On évite ainsi le risque de continuer à stimuler les jambes lorsque le véhicule avance à grande vitesse, par exemple à la descente.

Les stimulations électriques sont avantageusement stoppées dès réception d'un signal provenant d'un capteur de freinage. On évite ainsi le risque de continuer à stimuler les jambes lorsque l'utilisateur veut freiner.

Les stimulations électriques dépendent d'un signal délivré par un capteur mesurant l'inclinaison du véhicule. On peut ainsi adapter la stimulation, par exemple pour exercer plus de puissance par les membres inférieurs à la montée et pour exercer moins de puissance à la descente.

La méthode peut comprendre une étape de réduction de la stimulation des membres inférieurs en cas de détection d'une réduction de la puissance volontaire fournie par les membres inférieurs, par exemple en cas de fatigue.

Le véhicule à pédales est adapté pour se déplacer le long d'un parcours au gré des envies de l'utilisateur du véhicule. Il convient aussi pour une utilisation statique similaire à un appareil stationnaire. Dans ce dernier cas, le véhicule à pédales peut par exemple être monté sur un mécanisme destiné à désengager les roues du sol.

Selon une forme d'exécution, la stimulation électrique des membres inférieurs est déterminée en fonction notamment d'un facteur de stimulation représentant un pourcentage de la puissance délivrée intentionnellement par un ou plusieurs membres valides de l'utilisateur et en tenant compte de la puissance développée intentionnellement par ledit au moins un membre destiné à être stimulé lorsque celui-ci n'est pas stimulé.

Selon une forme d'exécution, le module de commande transmet des stimulations électriques de sorte à stimuler au moins un membre inférieur afin que celui-ci développe une puissance qui s'approche ou atteint une valeur de puissance cible.

La valeur de puissance cible correspond au produit du facteur de stimulation et de la puissance délivrée intentionnellement par le ou les membres supérieurs. La valeur de puissance cible est donc adaptée en temps réel en fonction de la valeur de la puissance délivrée intentionnellement par un ou plusieurs membres valides de l'utilisateur.

Selon une forme d'exécution avantageuse, la méthode de stimulation électrique comporte les étapes suivantes :
- entrer un programme d'entrainement et une valeur de puissance cible, via une interface utilisateur, la valeur de puissance cible correspondant à la puissance que doit développer le ou les membres inférieurs stimulés, et
- commander la ou les électrodes de stimulation de sorte à stimuler ledit membre stimulé afin de s'approcher ou d'atteindre la valeur cible.

Selon une forme d'exécution avantageuse, la méthode comprend une détermination de la puissance développée intentionnellement par les membres inférieurs, c'est-à-dire la puissance développée par ces membres qui ne dépend pas de la stimulation électrique.

Cette détermination de la puissance développée intentionnellement par les membres inférieurs peut être effectuée par exemple lors d'une phase de calibration, l'utilisateur actionnant ses membres inférieurs sans stimulation.

Cette détermination de la puissance développée intentionnellement par les membres inférieurs peut être effectuée en cours d'utilisation avec les membres inférieurs stimulés, lorsque l'on connaît la puissance additionnelle délivrée par chaque membre lorsqu'il est stimulé. En mesurant la puissance totale délivrée, et en y soustrayant cette puissance obtenue grâce à la stimulation, on peut ainsi déterminer la puissance délivrée intentionnellement par chaque membre inférieur.

La détermination de la puissance développée intentionnellement par les membres inférieurs peut aussi être effectuée par approximation, par exemple évaluée par un coach ou mesurée à l'aide d'autres exercices.

La puissance développée intentionnellement est de préférence stockée dans une mémoire.

En connaissant (au moins approximativement) la puissance développée intentionnellement par les membres inférieurs, on peut déterminer plus directement la valeur du signal de stimulation à appliquer pour produire la puissance totale désirée. Ainsi, lorsque la puissance totale souhaitée en un instant donné est inférieur à la puissance que l'utilisateur peut développer intentionnellement, le dispositif peut uniquement déterminer qu'aucune stimulation n'est nécessaire ; on évite ainsi d'appliquer une stimulation et de mesurer son effet avant de se rendre compte qu'elle n'était pas utile. Le dispositif peut ainsi appliquer en tout temps la stimulation qui est nécessaire pour développer la puissance totale souhaitée, en réduisant le risque de sur-stimuler initialement un muscle que l'utilisateur est déjà en train d'actionner intentionnellement.

Un second aspect de l'invention porte sur un véhicule à pédales, pour utilisateur, comportant un châssis, un siège soutenu par le châssis, au moins deux roues agencées respectivement à l'avant et à l'arrière du siège, une direction, un premier organe d'entrainement adapté pour être actionné par les membres supérieurs et/ou par les membres inférieurs de l'utilisateur, un second organe d'entrainement adapté pour être actionné par les membres inférieurs et/ou les membres supérieurs de l'utilisateur, au moins un frein et un dispositif de stimulation électrique.

Le dispositif de stimulation électrique comporte un module de commande, un module de mesure de données générées par l'utilisateur et au moins une électrode. L'électrode peut être appliquée directement sur au moins l'un des membres de l'utilisateur ou indirectement, par exemple sur la moelle épinière ou sur un nerf de l'utilisateur. Un programme informatique est stocké dans une mémoire du module de commande. Ce programme informatique est configuré pour exécuter la méthode et de ses différentes formes d'exécution décrites ci-dessus.

Selon une forme d'exécution, le module de mesure de données comporte un capteur de force ou de puissance agencé sur au moins un des premier et second organes d'entrainement afin de transmettre un signal au module de commande qui est proportionnel à la force respectivement à la puissance exercée sur le ledit au moins un des premier et second organes d'entrainement par au moins un des membres de l'utilisateur.

Selon une forme d'exécution, le module de mesure de données comporte un capteur angulaire agencé sur au moins un des premier et second organe d'entrainement afin de transmettre un signal au module de commande relatif à la position angulaire des manivelles du premier ou du second organe d'entrainement et ainsi de synchroniser les stimulations électriques en fonction de la position angulaire de l'un des premiers et second organe d'entrainements.

Selon une forme d'exécution, le module de commande comporte une première unité de traitement pour traiter des données relatives à l'intention de l'utilisateur, une seconde unité de traitement pour traiter des données relatives au membre stimulé, et une unité de gestion. L'unité de gestion est configurée pour transmettre à ladite au moins une électrode des stimulations électriques en fonction des données transmises par les deux unités de traitement.

Selon une forme d'exécution, l'unité de gestion est programmable de sorte à pouvoir entrer un programme d'entrainement comportant une valeur de puissance cible à appliquer sur l'un des premier et second organes d'entrainement par au moins un membre stimulé par l'électrode.

Selon une forme d'exécution, le véhicule à pédales comporte en outre une unité d'affichage permettant d'afficher la valeur de puissance cible et la valeur de la puissance exercée par ledit au moins un membre stimulé sur l'un des premier et second organes d'entrainement. La valeur de la puissance exercée par le membre stimulé peut être affichée de sorte à ce que la valeur de la puissance résultante de la stimulation électrique soit dissociée de la valeur de la puissance exercée intentionnellement par l'utilisateur afin de renseigner ce dernier sur son effort personnel sur le membre stimulé. L'unité d'affichage est par exemple réalisée sous la forme d'un smartphone, d'une tablette numérique, d'une montre connectée ou de lunettes de réalité augmentée.

Selon une forme d'exécution, le dispositif électrique de stimulation comporte un moyen de réglage manuel pour régler l'intensité du signal de stimulation transmis à ladite au moins une électrode. Le moyen de réglage manuel est de préférence sous la forme d'un potentiomètre ou d'un organe d'entrée de commande agencé pour permettre uniquement de réduire l'intensité du signal de stimulation.

Un troisième aspect de l'invention porte sur une application informatique téléchargeable sur un smartphone ou sur une tablette numérique. L'application informatique est configurée pour exécuter la méthode et de ses différentes formes d'exécution décrites ci-dessus. L'application informatique peut en outre permettre non seulement la sauvegarde des valeurs mesurées, notamment par les capteurs de force ou de puissance, mais aussi l'analyse de ces valeurs afin de pouvoir adapter les caractéristiques de la stimulation électrique en fonction de ces données. Le module de commande fait partie de l'électronique du smartphone, de la tablette numérique, de la montre connectée ou des lunettes de réalité augmentée selon une forme d'exécution avantageuse de l'invention.

Le terme de force est employé à de nombreux endroits dans la présente demande. L'homme du métier comprendra que dans le cas d'une rotation, la détermination ou la mesure de la force implique aussi une détermination ou mesure d'un couple, les deux étant équivalents lorsque le bras de levier est fixe et connu. Par simplification, la description et les revendications parlent cependant de mesure ou de détermination de force.

### Brève description des figures

Des exemples de mise en oeuvre de l'invention sont indiqués dans la description illustrée par les figures annexées dans lesquelles :
- La figure 1 représente une vue en perspective d'un véhicule à pédales selon une forme d'exécution préférentielle de l'invention ;
- La figure 2 représente une vue en perspective du pédalier de la figure 1;
- La figure 3 représente une vue de côté d'un véhicule à pédales similaire au véhicule à pédales selon la figure 1 ;
- Les figures 4a et 4b représentent des exemples de diagrammes circulaires selon respectivement un premier et un second mode de stimulation électrique appliquée à l'utilisateur du véhicule à pédale ;
- La figure 5 est un schéma blocs illustrant le type d'information traitées par le contrôleur pour stimuler les électrodes ;
- La figure 6 est une représentation graphique de la force ou puissance développée en fonction de position angulaire d'une manivelle, le diagramme illustrant la valeur de cible sur le pied gauche, la valeur mesurée sur ce pied, et la contribution due à la stimulation du muscle correspondant.
- La figure 7 représente un schéma-bloc du contrôleur di dispositif;
- La figure 8 est un graphique représentant un exemple d'évolution dans le temps de la stimulation nécessaire pour maintenir une puissance cible constante ;
- Les figures 9a à 9c sont des graphiques représentant la puissance mesurée d'au moins un membre valide (barre de gauche) et d'au moins un membre invalide (barre de droite), la proportion entre la puissance développée intentionnellement et la puissance due à la stimulation du membre invalide variant selon ces exemples.
- La figure 9d est un graphique similaire aux figures 9a à 9c, la valeur de puissance cible étant de 25% de la valeur de la puissance mesurée sur un membre valide, et
- La figure 10 est un graphique représentant l'évolution de la valeur de puissance cible en fonction de la valeur de la puissance mesurée sur un membre valide au cours d'un intervalle de temps donné.

### Exemples de mode de réalisation de l'invention

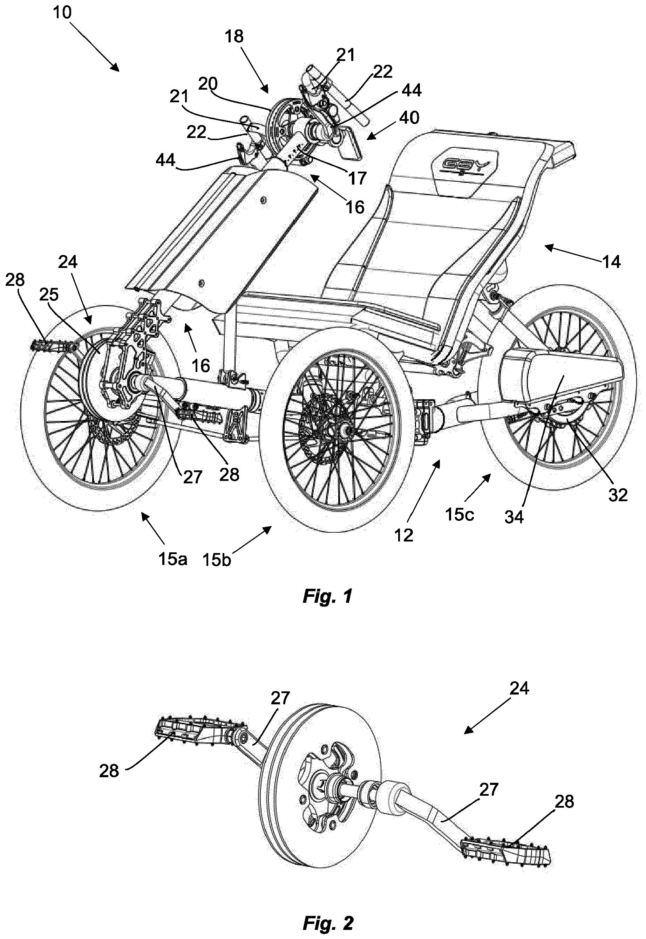

Selon une forme de réalisation illustrée en particulier à la figure 1, le véhicule à pédales 10 est adapté pour la rééducation d'une personne ayant une mobilité réduite, par exemple d'une personne possédant un ou plusieurs membres totalement ou partiellement paralysés ou d'une personne ayant au moins une perte partielle de la motricité d'un ou plusieurs membres. Le véhicule à pédales 10 selon l'invention est particulièrement bien adapté pour la rééducation d'une personne ayant perdu l'usage de ses membres inférieurs ou tout du moins ayant une perte partielle de la motricité de ses membres inférieurs alors que ses membres supérieurs sont toujours valides. Le véhicule peut toutefois être adapté pour une personne ayant subi un AVC et qui a perdu l'usage de ses membres inférieur et supérieur droits ou de ses membres inférieur et supérieur gauches ou tout du moins une perte partielle de la motricité de ces membres. Ce véhicule peut également être adapté pour une personne amputée. Le véhicule à pédales 10 peut en outre être utilisé pour une activité sportive pour les personnes limitées dans leur mobilité.

A cet effet, selon la figure 1, le véhicule à pédales 10 comporte un châssis 12, un siège 14 soutenu par le châssis 12, deux roues avant 15a, 15b et au moins une roue arrière 15c montées sur le châssis 12 de part et d'autre du siège 14 et une direction 16. Le véhicule à pédales peut toutefois comporter deux roues avant et deux roues arrière ou uniquement une roue avant et deux roues arrière selon deux variantes d'exécution non-illustrées. Un premier et un second organe d'entrainement 18, 24 du véhicule à pédales 10 sont montés aux extrémités respectives d'une colonne de direction 17 et sont reliés cinématiquement par une transmission agencée sur la colonne de direction 17 de manière à lier les deux pédaliers, par exemple par une transmission du type chaîne ou courroie.

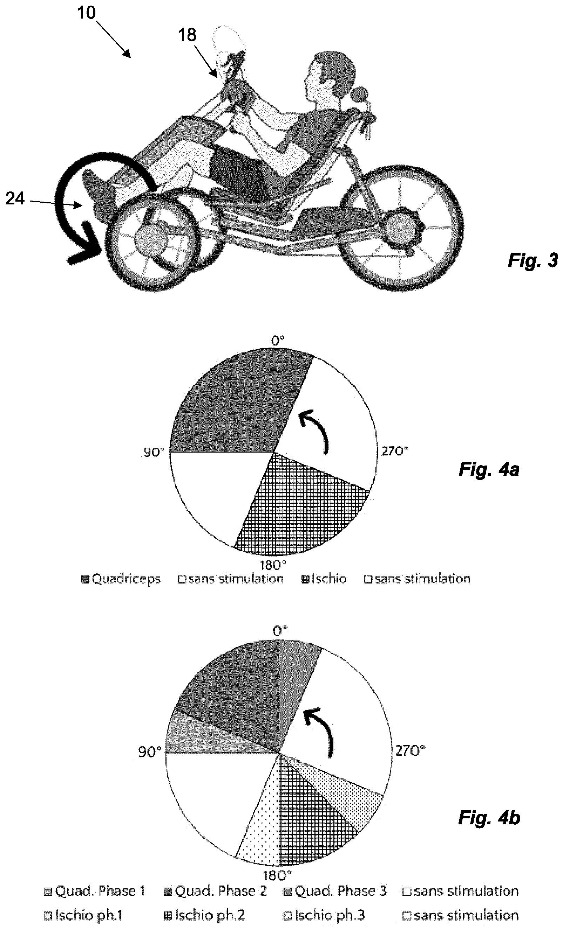

Le premier organe d'entrainement 18 est actionnable par les membres supérieurs de l'utilisateur alors que le second organe d'entrainement 24 est actionnable par les membres inférieurs de l'utilisateur comme illustré à la figure 3. A cet effet, selon la figure 1, le premier organe d'entrainement 18 comporte un premier engrenage d'entrainement 20 actionnable en rotation par deux poignées 22 agencées de part et d'autre de l'engrenage 20 et reliées à celui-ci par deux manivelles 21. Le second organe d'entrainement 24 comporte un second engrenage d'entrainement 25 actionnable en rotation par deux pédales 28 agencées de part et d'autre de l'engrenage 25 et reliées à celui-ci par deux manivelles 27 (figure 2).

Dans le mode de réalisation illustré, les premiers et deuxièmes organes d'entraînement entraînent la roue arrière 15c du véhicule au travers d'une transmission comportant par exemple une chaîne reliant le second organe d'entrainement 24 à un moyeu 32 monté sur la roue arrière 15c du véhicule. Une courroie ou tout autre type de transmission à la place de la chaîne peut être implémenté selon une variante. Dans une variante non représentée, il est aussi possible d'entraîner les roues avant du véhicule avec les premiers et deuxièmes organes d'entraînement.

Le véhicule à pédales 10 comporte par ailleurs une cassette et un dérailleur (non visible) pour adapter les rapports d'engrenage en fonction du profil du parcours emprunté par l'utilisateur du véhicule à pédales 10.

Dans une option, le moyeu 32 peut être entraîné par un moteur électrique optionnel alimenté par une batterie 34. Dans une variante d'exécution, le véhicule à pédale peut être dépourvu de moteur électrique et comporter uniquement un engrenage comprenant la cassette et le dérailleur.

La cassette et le dérailleur du véhicule à pédales 10, avec ou sans moteur électrique, peuvent être remplacé par tout autre système équivalent de démultiplication de la force.

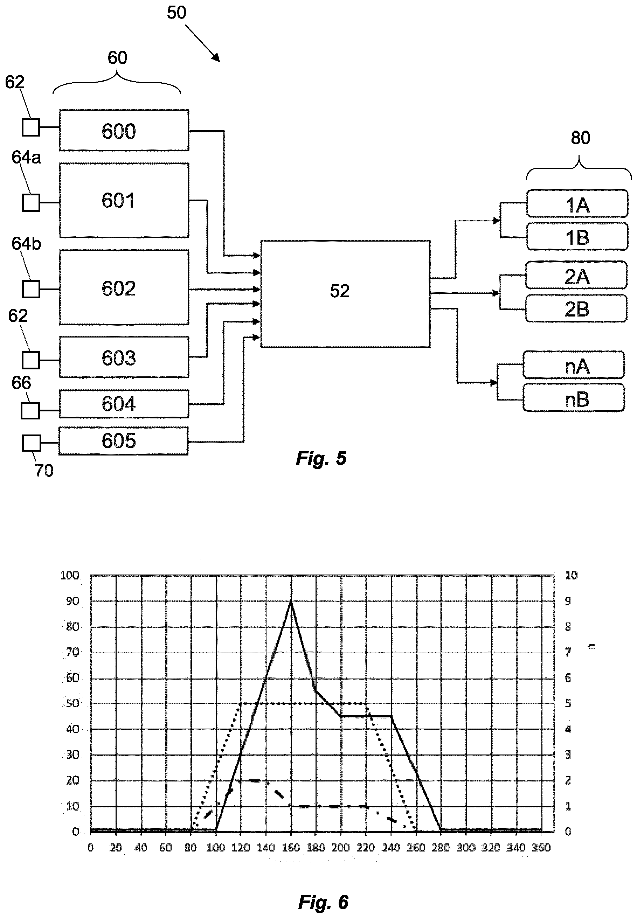

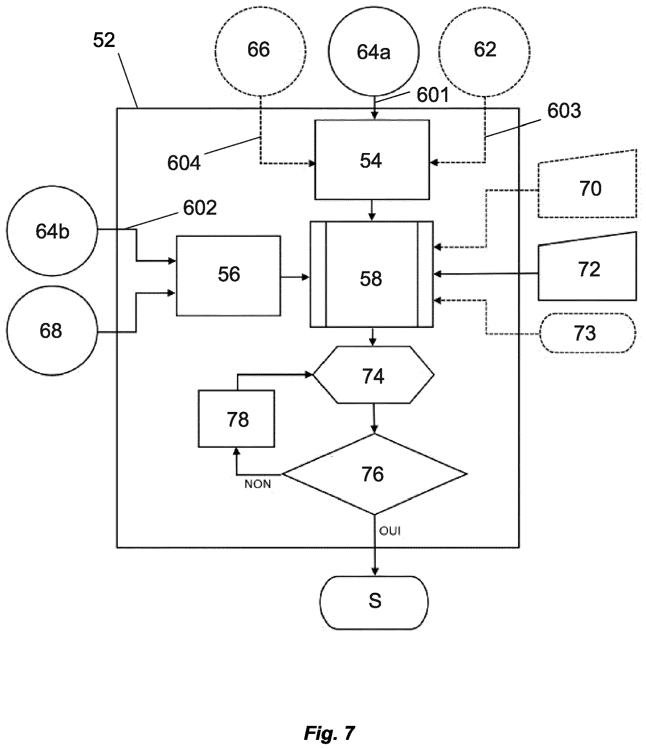

Selon l'invention et en référence aux figures 5 et 7, le véhicule à pédales 10 comporte en outre un dispositif de stimulation électrique 50. Celui-ci comporte un module de commande 52, un module de mesure de données 60 et une ou plusieurs électrodes 80.

Le module de mesure de données 60 est destiné à mesurer des données indicatrices de l'intention de l'utilisateur ou d'autres données dépendant des actions de l'utilisateur. Ces données sont générées par différents capteurs équipant le véhicule à pédales 10.

Les électrodes 1A à nB (globalement électrodes 80) peuvent se présenter sous différentes formes, par exemple sous la forme de patchs adaptés pour être appliqués sur au moins un des membres de l'utilisateur afin de stimuler le membre en question, sous la forme d'électrodes sous-cutanées pour une stimulation nerveuse périphérique (PNS) ou sous la forme d'une électrode positionnée sur la moelle épinière pour une stimulation électrique épidurale.

De manières avantageuses, les électrodes 80 peuvent par exemple être appliquées sur les deux membres inférieurs d'un utilisateur du véhicule à pédale ayant perdu l'usage de ses membres inférieurs ou tout du moins ayant une perte partielle de la motricité de ses membres inférieurs alors que ses membres supérieurs sont toujours valides.

Selon la figure 7, le module de commande 52 comporte de préférence un microcontrôleur avec une première unité de traitement 54 pour traiter des données relatives à l'intention de l'utilisateur, en particulier les données 601 relatives à la force ou à la puissance exercée intentionnellement par les membres valides de l'utilisateur telles que mesurées par un capteur de force ou de puissance 64a. L'unité de traitement 54 reçoit aussi un signal de freinage 604 d'un capteur 66 détectant l'actionnement d'un frein, et de préférence un signal d'un capteur ou module de détection du sens du mouvement. L'unité de traitement 54 peut en outre recevoir un signal 600 d'un capteur ou d'un encodeur 62 permettant de déterminer la position angulaire du moyeu du premier organe d'entraînement 18. D'autres capteurs, par exemple un inclinomètre non représenté pour mesurer l'inclinaison du véhicule, peuvent être prévus et traités par l'unité de traitement 54.

Une seconde unité de traitement 56 traite les données relatives à un membre ou plusieurs membres stimulés de l'utilisateur, par exemples les deux membres inférieurs, en recevant un signal 602 d'au moins un capteur de force ou de puissance 64b et de préférence un signal d'au moins un capteur 68 de position angulaire du moyeu du deuxième organe d'entraînement 24.

Une unité de gestion 58 est configurée pour transmettre aux électrodes 80 des stimulations électriques en fonction des données transmises par les deux unités de traitement 54, 56.

Les électrodes 80 peuvent par exemples être placées sur les muscles ischio-jambier et sur les muscles quadriceps des deux jambes de l'utilisateur du véhicule. Selon une variante, les électrodes peuvent être directement implantées dans la moelle épinière de l'utilisateur. Le signal électrique transmis aux électrodes 80 peut être adapté en modifiant par exemple l'amplitude et/ou la fréquence du signal, la longueur de signal, la forme du signal ou la polarité du signal. Une combinaison successive régulière ou non de plusieurs séquences de stimulation électrique peut également être appliquée aux muscles des deux jambes de l'utilisateur comme illustrée par les figures 4a et 4b.

Ces figures illustrent à titre d'exemples des séquences de stimulations électriques afin de stimuler les muscles adéquates pour actionner en rotation les organes d'entrainement 18, 24 du véhicule à pédale.

Les données relatives à l'intention de l'utilisateur sont acquises par le module de mesure de données 60. Celui-ci comporte un capteur ou un arrangement de capteurs destinés à mesurés différents paramètres. Par exemple, un premier capteur de force ou de puissance 64a peut être agencé sur au moins une poignée 22 du premier organe d'entrainement 18 pour mesurer la force, et en déduire si nécessaire la puissance exercée par un membre valide de l'utilisateur alors qu'un second capteur de force 64b est agencé sur au moins une pédale 28 du second organe d'entrainement 24 pour mesurer la force exercée par un membre stimulé de l'utilisateur (et en déduire si nécessaire la puissance). Alternativement, un capteur de couple peut être placé dans l'axe des manivelles du premier organe d'entrainement 18 et/ou du second organe d'entrainement 24.

La détermination de la puissance exercée par les membres inférieurs respectivement supérieure peut être effectuée par le contrôleur 52 en multipliant le couple obtenu par la vitesse de rotation angulaire.

Un capteur angulaire 62 peut également être agencé pour mesurer la position angulaire des manivelles des premier et second organe d'entrainement 18, 24 de sorte à synchroniser les stimulations électriques en fonction de la position angulaire de l'un des premiers et second organe d'entrainements.

Selon une forme avantageuse d'exécution, la stimulation électrique 74 appliquée par les électrodes 80 grâce au dispositif de stimulation électrique 50 dépend de la puissance ou de la force exercée intentionnellement par les membres supérieurs valides. Ainsi, lorsque l'utilisateur actionne ses bras avec plus de puissance (par exemple avec plus de force ou plus rapidement), la stimulation électrique appliquée sur les membres inférieurs est adaptée. Cela permet par exemple de solliciter davantage les jambes lorsque les bras exercent une puissance plus importante, par exemple à la montée ou lors d'une accélération, et inversement de réduire la stimulation des jambes lorsque les bras développent moins de puissance, par exemple à la descente ou lors d'une décélération.

Lorsque, comme dans l'exemple illustré, les premiers et deuxièmes organes d'entraînement 18, 24 sont reliés l'un à l'autre par une transmission mécanique, une mesure de la force exercée par les bras et de celle exercée par les jambes est suffisante ; en effet la vitesse de rotation de bras et des jambes est alors identique, ou en tout cas dans un rapport constant, en sorte qu'une force des jambes proportionnelle à celle des bras garantit aussi des puissances proportionnelles.

Lorsque toutefois les premiers et deuxièmes organes d'entraînement 18, 24 sont découplés ou reliés l'un à l'autre via un dérailleur par exemple, la vitesse de rotation des premiers et deuxièmes organes d'entraînement est variable. Une force développée par les jambes proportionnelle à celle développée par les bras ne garantit alors plus que les puissances soient aussi proportionnelles. Il est alors souhaitable de déterminer la puissance développée par les membres supérieurs pour contrôler la puissance développée par les membres inférieurs. Contrôler la puissance développée par les membres inférieurs permet par exemple d'éviter une sur-sollicitation pouvant provoquer une fatigue ou même des risques de blessure.

La puissance développée par les membres inférieurs comporte une puissance provoquée par la stimulation électrique et une puissance développée intentionnellement, dans le cas par exemple d'une paraplégie partielle. La stimulation des membres inférieurs est donc effectuée en tenant compte de cette puissance développée intentionnellement, hors sollicitation, afin que la puissance totale développée par les membres inférieurs se trouve dans la proportion voulue avec la puissance développée par les membres supérieurs. Si la puissance développée intentionnellement par les jambes est connue, il est possible d'appliquer immédiatement un signal de stimulation adapté afin de développer la puissance totale recherchée, sans avoir besoin d'appliquer initialement une stimulation générant une puissance excessive qui doit ensuite être réduite pour tenir compte de la puissance intentionnelle.

La stimulation électrique peut être adaptée pour des personnes souffrant d'une hémiplégie suite à un accident vasculaire cérébral. Dans ce cas, la force peut par exemple être mesurée par des capteurs de force se trouvant sur l'une des poignées 22 du premier organe d'entrainement 18 et sur l'une des pédales 28 de second organe d'entrainement 24 se trouvant sur le même coté.

Ceci a pour avantage de permettre une stimulation électrique des muscles ou des nerfs de l'utilisateur qui est, en tout temps, en phase avec ses capacités et son intention de pédalage.

Selon la figure 7, l'unité de gestion 58 du module de commande 52 est programmable de sorte à pouvoir entrer des valeurs 72 programmables selon l'utilisateur, par exemple des valeurs cibles ou un programme d'entrainement pouvant être sélectionné, via une interface utilisateur, par l'utilisateur lui-même ou par un thérapeute. L'interface utilisateur permet également d'entrer une valeur cible de la puissance ou de la force résultante sur l'un des premier et second organes d'entrainement 18, 24 par au moins un membre stimulé par l'électrode 80. Au cours d'un entrainement selon le programme sélectionné, l'unité de gestion 58 détermine si la valeur de puissance cible correspond à la valeur de la puissance exercée par un membre stimulé sur l'un des premier et second organes d'entrainement 18, 24 et adapte la stimulation électrique sur le membre stimulé tant que la puissance mesurée n'atteint pas la valeur cible, pour autant que l'intensité de la stimulation électrique n'excède pas une valeur limite préalablement fixée, par exemple par un thérapeute.

Au cours de l'utilisation du véhicule à pédale, la valeur de puissance cible est ajustée en temps réel en fonction de la mesure de la puissance exercée par les membres valides de l'utilisateur et d'un facteur de stimulation. Le facteur de stimulation représente un pourcentage de la puissance développée intentionnellement par les membres valides de l'utilisateur. Ce facteur de stimulation est initialement déterminé et programmé, par l'utilisateur ou par un thérapeute, au cas par cas en fonction des capacités de l'utilisateur du véhicule à pédales. Le facteur de stimulation peut par exemple être programmé à 2:1 pour une personne dont la capacité motrice des membres inférieure est modérément diminuée ou par exemple à 4:1 pour une personne dont la capacité motrice est plus sévèrement diminuée. Le facteur de stimulation peut être ajusté par le module 78 (figure 7) par exemple lorsque la puissance totale mesurée ne correspond pas à la valeur cible (test 76). Le signal de stimulation corrigé est illustré par la référence s.

Dans un exemple, les membres supérieurs développent une puissance instantanée de 100 Watts. Si le facteur de stimulation est de 2 :1, les membres inférieurs doivent développer une puissance de 50 Watts. Dans le cas où la puissance intentionnelle développée par les membres inférieurs lorsqu'aucune stimulation n'est appliquée est de 20 Watts, la stimulation sera déterminée de manière à produire une puissance additionnelle de 30 Watts. La puissance totale développée par les membres inférieurs est alors bien de 50 Watts.

La puissance intentionnelle développée par les membres inférieure est une donnée qui peut être par exemple stockée par le contrôleur 52. Elle peut être déterminée empiriquement par le thérapeute, mesurée lors d'une phase de calibration en demandant à l'utilisateur de pédaler avec les membres inférieurs sans stimulation, et/ou mesurée en cours d'utilisation lorsque la puissance obtenue grâce à la stimulation est connue.

La figure 6 est une représentation graphique de la puissance ou force exercée par un membre (ici le pied gauche) en fonction de la position angulaire de la manivelle. Dans cet exemple, la force ou la puissance en ordonnée (trait plein) exercée par le pied gauche du membre stimulé de l'utilisateur du véhicule à pédales est mesurée et le module de commande du dispositif de stimulation électrique adapte la stimulation électrique afin que les muscles du membre stimulé, par exemple le muscle ischio-jambier en alternance avec le muscle quadriceps, se contractent suffisamment pour atteindre la valeur de puissance cible en pointillé. Le trait mixte illustre la puissance ou la force due à la stimulation du muscle.

Le facteur de stimulation peut en tout temps être modifié en prenant en compte toutes les données pouvant avoir un impact sur la fréquence de pédalage de l'utilisateur et qui nécessitent une adaptation de la stimulation électrique appliquée au membre invalide ou partiellement invalide. Ces données peuvent par exemple être relatives à la fréquence cardiaque, à la consommation maximale d'oxygène (VO₂max) ou à la fatigue musculaire de l'utilisateur.

Dans un exemple, une fatigue des membres inférieure est détectée lorsque la stimulation qui doit être appliquée pour que les muscles des membres inférieurs développent une puissance donnée augmente ; cela signifie que la puissance additionnelle obtenue grâce à la stimulation augmente et donc que la puissance développée intentionnellement diminue. Dans un tel cas, l'unité 52 réduit avantageusement la stimulation électrique, par exemple en adaptant le facteur de stimulation, pour éviter une fatigue excessive.

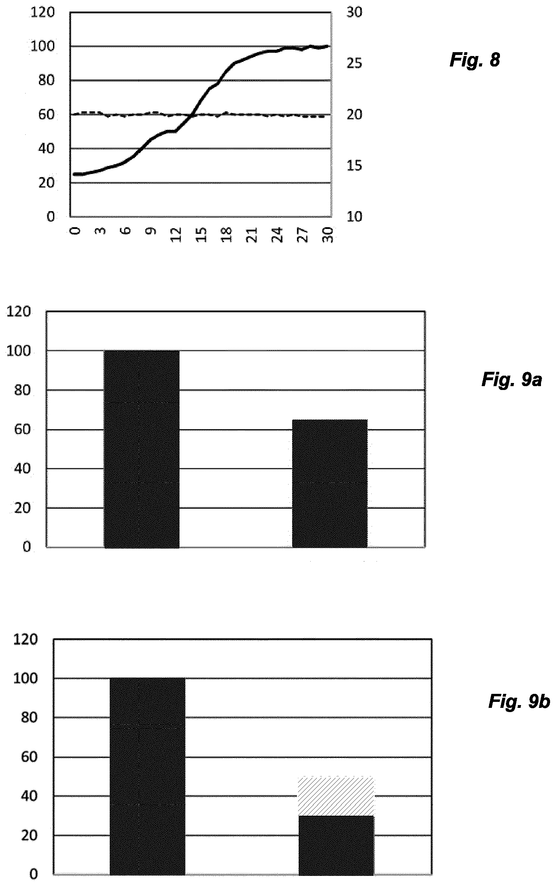

Selon la figure 8, la valeur de puissance cible (ligne traitillée) peut être maintenue constante et la stimulation électrique (ligne pleine) adaptée en fonction de la participation musculaire des membres stimulés, de sorte que la puissance de pédalage sans application de force volontaire corresponde à la valeur cible sur une période déterminée. L'objectif est de connaître la fatigue musculaire générée par la stimulation afin de pouvoir déterminer un facteur de stimulation prenant en compte cette fatigue musculaire. Le facteur de stimulation peut également être déterminé en appliquant une stimulation constante et en mesurant la diminution de puissance développée par le membre stimulé.

La stimulation des membres inférieure peut aussi être adaptée en fonction de la vitesse de rotation et/ou de l'inclinaison du véhicule. Par exemple, lorsque le véhicule se déplace rapidement à faible puissance, par exemple à la descente, et/ou que l'inclinomètre détecte que le véhicule descend, la stimulation électrique des membres inférieure peut être réduite. La stimulation électrique peut ainsi être diminuée, voire même supprimée lorsque le véhicule se trouve dans une descente. A l'inverse, cette stimulation peut être augmentée à la montée ; il est même possible d'augmenter le facteur de stimulation à la montée afin d'augmenter la proportion dans laquelle les jambes contribuent à l'effort à la montée.

La valeur de puissance cible peut par ailleurs être modifiée en fonction des données transmise par un capteur de freinage 66 afin de diminuer, voire même de supprimer la stimulation électrique en fonction de la force appliquée sur le frein 44.

Selon la figure 1, le véhicule à pédales 10 peut comporter en outre, selon une forme d'exécution avantageuse, une unité d'affichage 40 (module 73 sur la figure 7) faisant office d'interface utilisateur et permettant d'entrer la valeur cible et d'afficher la valeur cible et la valeur de la force exercée par au moins un membre stimulé sur l'un des premier et second organes d'entrainement 18, 24. L'unité d'affichage 40 peut par exemple être l'écran d'un smartphone ou d'une tablette numérique qui peuvent être montés sur un support adapté (non visible). L'unité d'affichage 40 peut également être l'écran d'une montre connectée ou les verres de lunettes de réalité augmentée portée(s) par l'utilisateur du véhicule à pédales.

Les figures 9a-9c illustrent différents exemples d'entrainement. Selon la figure 9a, la stimulation électrique est calculée sur la base d'un facteur de stimulation 2:1. La puissance exercée de manière volontaire (portion en noir de la barre de droite) par un membre dont la motricité est limitée, est ici de 65% de la puissance exercée par le membre valide (barre de droite), soit au-dessus de 50%. Le microcontrôleur 7 détecte immédiatement qu'aucune stimulation électrique n'est nécessaire.

Selon la figure 9b, la stimulation électrique est également calculée sur la base d'un facteur de stimulation 2:1. Dans cet exemple, la puissance exercée de manière volontaire par le membre dont la motricité est limitée (portion en noir de la barre de droite) est seulement de 30% de la puissance développée par le membre valide (barre de gauche), soit inférieure à 50%. Le module de commande du dispositif de stimulation électrique va donc envoyer un signal adéquat aux électrodes 80 afin de stimuler le membre en question de sorte à atteindre la valeur cible. La stimulation électrique permet donc, selon cet exemple, d'augmenter la capacité motrice de 20% (puissance additionnelle hachurée sur la barre de droite) pour atteindre les 50% de la puissance exercée par les membres valides de l'utilisateur (barre de gauche).

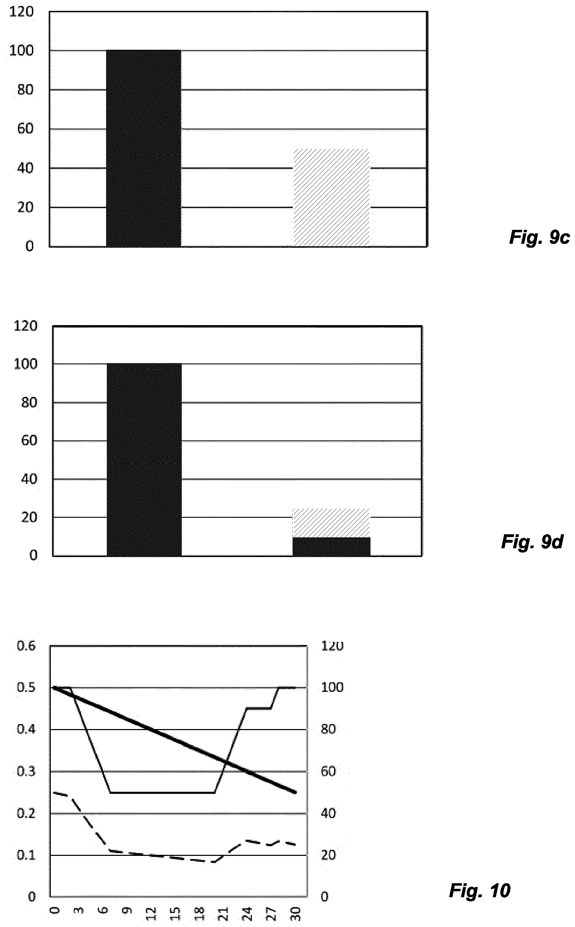

Selon la figure 9c, la stimulation électrique est également calculée sur la base d'un facteur de stimulation 2:1. Dans cet exemple, aucune force volontaire n'est générée par le membre invalide (barre de droite). Le module de commande du dispositif de stimulation électrique va donc envoyer un signal adéquat aux électrodes afin de stimuler le membre en question de sorte à atteindre la valeur cible de 50% de la puissance exercée par les membres valides de l'utilisateur.

Selon la figure 9d, la stimulation électrique est calculée sur la base d'un facteur de stimulation 4:1. Dans cet exemple, la puissance exercée de manière volontaire par le membre dont la motricité est limitée est seulement d'environ 10%, soit inférieure à 25%. Le module de commande du dispositif de stimulation électrique va donc envoyer un signal adéquat aux électrodes afin de stimuler le membre en question de sorte à atteindre la valeur cible de 25% de la puissance exercée les membres valides de l'utilisateur (puissance additionnelle obtenue grâce à la stimulation en hachuré sur la barre de droite).

Dans l'exemple de la figure 10, le facteur de stimulation (ligne pleine) passe d'un rapport de 2:1 à un rapport de 4:1 dans un intervalle de temps prédéterminé afin de prendre en compte notamment la fatigue musculaire de l'utilisateur.

Une application informatique peut être téléchargée et exécutée par le smartphone, la tablette numérique ou la montre connectée afin de commander le dispositif de stimulation électrique. L'application informatique peut par ailleurs être configurée pour proposer différents programmes d'entrainement. Selon ce mode d'exécution, l'électronique du Smartphone, de la tablette numérique, de la montre connectée ou de tout autre unité électronique réalise la fonction du module de commande du dispositif de stimulation électrique pour traiter les données qui sont transmises par le module de mesure 60.

Le dispositif de stimulation électrique 50 peut comporter un moyen de réglage manuel 70 de la stimulation électrique de sorte que l'utilisateur de véhicule à pédales puisse diminuer la stimulation électrique en fonction de son ressenti. Un potentiomètre peut par exemple remplir la fonction de réglage manuel.

### Références

***Véhicule à pédales 10***
*Châssis 12*
*Siège 14*
*Roues avant 15a, 15b*
*Roue arrière 15c*
*Direction 16*
   *Colonne de direction 17*
*Premier organe d'entrainement 18*
   *Premier engrenage d'entrainement 20*
   *Manivelles 21*
   *Poignées 22*
*Second organe d'entrainement 24*
   *Second engrenage d'entrainement 25*
   *Manivelles 27*
   *Pédales 28*
*Transmission*
   *Chaine*
   *Moteur (premier mode d'exécution)*
   *Moyeu électrique 32*
      *Cassette (Second mode d'exécution)*
      *Dérailleur*
   *Batterie 34 (premier mode d'exécution)*
*Unité d'affichage 40*
   *Smartphone*
   *Support*
*Frein à main 44*
***Dispositif de stimulation électrique 50***
   *Module de commande 52*
      *Microcontrôleur*
      *Première unité de traitement 54*
      *Seconde unité de traitement 56*
      *Unité de gestion 58*
   *Module de mesure de données 60*
      *Capteur angulaire 62*
      *Capteur de force 64a, 64b*
      *Capteur de freinage 66*
   *Dispositif de réglage manuel 70*
      *Potentiomètre*
   *Electrodes 80*
      *Electrode patchs*

## Revendications

1. **Véhicule à pédales** (10) destiné à se déplacer le long d'un parcours au gré des envies d'un utilisateur, comportant un châssis (12), un siège (14) soutenu par le châssis (12), au moins deux roues (15a, 15b, 15c) agencées respectivement à l'avant et à l'arrière du siège (14), une direction (15), un premier organe d'entrainement (18) adapté pour être actionné par les membres supérieurs de l'utilisateur, un second organe d'entrainement (24) adapté pour être actionné par les membres inférieurs de l'utilisateur, au moins un frein (44) et un dispositif de stimulation électrique (50) comportant un module de commande (52), un module de mesure de données (60) et au moins une électrode (80) adaptée pour stimuler au moins un membre inférieur destiné à être stimulé, le module de commande (52) étant configuré pour traiter des données relatives à l'intention de l'utilisateur et comportant un programme informatique configuré pour transmettre à ladite au moins une électrode (80) par le module de commande (52) des stimulations électriques en fonction des données relatives à l'intention de l'utilisateur,
**caractérisé en ce que** lesdites données relatives à l'intention de l'utilisateur comprennent la puissance ou la force développée intentionnellement par les membres supérieurs de l'utilisateur,
**en ce qu'**une étape de détermination de la puissance développée intentionnellement par les membres inférieurs est effectuée,
et **en ce que** lesdites stimulations électriques sont déterminées de manière à assurer une relation prédéterminée entre la puissance développée par les membres supérieurs et la puissance totale développée par les membres inférieurs, ladite puissance totale correspondant à la somme de la puissance développée intentionnellement par les membres inférieurs et de la puissance développée par les membres inférieurs grâce à la stimulation.

2. Véhicule à pédales (10) selon la revendication 1, **caractérisé en ce que** lesdites stimulations électriques (s) sont déterminées en fonction de la puissance respectivement de la force développée intentionnellement par les membres supérieurs de l'utilisateur et de la puissance respectivement de la force développée intentionnellement par les membres inférieurs de l'utilisateur.

3. Véhicule à pédales (10) selon la revendication 1 ou 2, **caractérisé en ce que** lesdites stimulations électriques sont déterminées de manière à assurer une proportion donnée entre la puissance développée intentionnellement par les membres supérieurs de l'utilisateur et la puissance totale développée par les membres inférieurs.

4. Véhicule à pédales (10) selon la revendication 1 à 3, **caractérisé en ce que** lesdites stimulations électriques sont stoppées ou réduites lorsque la puissance totale développée par un membre inférieur excède une valeur prédéterminée.

5. Véhicule à pédales (10) selon la revendication 1 à 4, **caractérisé en ce que** lesdites stimulations électriques sont stoppées ou réduites dès que la mesure de la vitesse de rotation de l'un des premier et second organes d'entrainement excède une vitesse de rotation prédéterminée.

6. Véhicule à pédales (10) selon la revendication 1 à 5, **caractérisé en ce que** lesdites stimulations électriques sont stoppées dès réception d'un signal provenant d'un capteur de freinage.

7. Véhicule à pédales (10) selon la revendication 1 à 6, **caractérisé en ce que** lesdites stimulations électriques dépendent d'un signal délivré par un capteur mesurant l'inclinaison du véhicule.

8. Véhicule à pédales (10) selon la revendication 1 à 7, **caractérisé en ce que** le programme informatique est en outre configuré pour réduire la stimulation des membres inférieurs en cas de détection d'une réduction de la puissance volontaire fournie par les membres inférieurs, par exemple en cas de fatigue.

9. Véhicule à pédales (10) selon l'une des revendications précédentes, **caractérisé en ce que** le module de mesure de données (60) comporte un capteur de force ou de couple (64a, 64b) agencé sur au moins un des premier et second organes d'entrainement (18, 24) de sorte à transmettre un signal au module de commande (52) qui dépend de la force exercée sur le ledit au moins un des premier et second organes d'entrainement (18, 24) par au moins un des membres de l'utilisateur.

10. Véhicule à pédales (10) selon la revendication précédente, **caractérisé en ce que** le module de mesure de données (60) comporte un capteur de vitesse de rotation agencé sur au moins un des premier et second organes d'entrainement (18, 24) de sorte à transmettre un signal au module de commande (52) proportionnel à la puissance exercée sur ledit au moins un des premier et second organes d'entrainement (18, 24) par au moins un des membres de l'utilisateur.

11. Véhicule à pédales (10) selon l'une des revendications précédentes, **caractérisé en ce que** le module de mesure de données (60) comporte un capteur angulaire (62) agencé sur au moins un des premier et second organe d'entrainement (18, 24) de sorte à transmettre un signal au module de commande (52) en relatif à la position angulaire de manivelles (21, 27) du premier ou du second organe d'entrainement (18, 24) de sorte à synchroniser les stimulations électriques en fonction de la position angulaire de l'un des premiers et second organe d'entrainements.

12. Véhicule à pédales (10) selon l'une des revendications précédentes, **caractérisé en ce que** le module de commande (52) comporte une première unité de traitement (54) pour traiter des données relatives à l'intention de l'utilisateur, une seconde unité de traitement (56) pour traiter des données relatives audit au moins un membre destiné à être stimulé et une unité de gestion (58) configurée pour transmettre à ladite au moins une électrode (80) des stimulations électriques en fonction des données transmises par les deux unités de traitement (54, 56).

13. Véhicule à pédales (10) selon la revendication précédente, **caractérisé en ce que** l'unité de gestion (58) est programmable de sorte à pouvoir entrer un programme d'entrainement comportant une valeur cible de la puissance totale exercée par les membres inférieurs.

14. Véhicule à pédales selon l'une des revendications précédentes, **caractérisé en ce que** la puissance exercée par le membre stimulé peut être affichée de sorte à ce que la valeur de la puissance résultante de la stimulation électrique soit dissociée de la valeur de la puissance exercée intentionnellement par l'utilisateur.

## Patentansprüche

1. **Pedalfahrzeug** (10) zum Bewegen entlang einer Strecke nach Belieben eines Nutzers, mit einem Rahmen (12), einem von dem Rahmen (12) getragenen Sitz (14), mindestens zwei Rädern (15a, 15b, 15c), die jeweils an der Vorder- und Rückseite des Sitzes (14) angeordnet sind, eine Lenkung (15), ein erstes Antriebselement (18), das so ausgebildet ist, dass es von den oberen Gliedmaßen des Nutzers betätigt werden kann, ein zweites Antriebselement (24), das so beschaffen ist, dass es von den unteren Gliedmaßen des Nutzers betätigt werden kann, mindestens eine Bremse (44) und eine elektrische Stimulationsvorrichtung (50) mit einem Steuermodul (52), einem Datenmessmodul (60) und mindestens einer Elektrode (80), die geeignet ist, mindestens eine untere Extremität zu stimulieren, die stimuliert werden soll, wobei das Steuermodul (52) so konfiguriert ist, dass es Daten bezüglich der Absicht des Nutzers verarbeitet, und ein Computerprogramm enthält, das so konfiguriert ist, dass es elektrische Stimulationen in Abhängigkeit von den Daten bezüglich der Absicht des Nutzers über das Steuermodul (52) an die mindestens eine Elektrode (80) überträgt,
**dadurch gekennzeichnet, dass** die Daten, die sich auf die Absicht des Nutzers beziehen, die Kraft oder Stärke umfassen, die absichtlich von den oberen Gliedmaßen des Nutzers entwickelt wird,
dass ein Schritt zur Bestimmung der absichtlich von den unteren Gliedmaßen entwickelten Leistung durchgeführt wird,
und dass die elektrischen Stimulationen so bestimmt sind, dass sie eine vorbestimmte Abhängigkeit zwischen der von den oberen Gliedmaßen entwickelten Leistung und der von den unteren Gliedmaßen entwickelten Gesamtleistung sicherstellen, wobei die Gesamtleistung der Summe der absichtlich von den unteren Gliedmaßen entwickelten Leistung und der von den unteren Gliedmaßen aufgrund der Stimulation entwickelten Leistung entspricht.

2. Pedalfahrzeug (10) nach Anspruch 1, **dadurch gekennzeichnet, dass** die elektrischen Stimulationen (s) in Abhängigkeit von der Leistung bzw. der Kraft, die absichtlich von den oberen Gliedmaßen des Nutzers entwickelt wird, und der Leistung bzw. der Kraft, die absichtlich von den unteren Gliedmaßen des Nutzers entwickelt wird, bestimmt werden.

3. Pedalfahrzeug (10) nach Anspruch 1 oder 2, **dadurch gekennzeichnet, dass** die elektrischen Stimulationen so bestimmt werden, dass ein gegebenes Verhältnis zwischen der absichtlich von den oberen Gliedmaßen des Nutzers entwickelten Leistung und der von den unteren Gliedmaßen entwickelten Gesamtleistung gewährleistet ist.

4. Pedalfahrzeug (10) nach Anspruch 1 bis 3, **dadurch gekennzeichnet, dass** die elektrischen Stimulationen unterbrochen oder verringert werden, wenn die von einer unteren Extremität entwickelte Gesamtleistung einen vorbestimmten Wert überschreitet.

5. Pedalfahrzeug (10) nach Anspruch 1 bis 4, **dadurch gekennzeichnet, dass** die elektrischen Stimulationen unterbrochen oder verringert werden, sobald die Messung der Drehgeschwindigkeit eines der ersten und zweiten Antriebselemente eine vorbestimmte Drehgeschwindigkeit überschreitet.

6. Pedalfahrzeug (10) nach Anspruch 1 bis 5, **dadurch gekennzeichnet, dass** die elektrischen Stimulationen unterbrochen werden, sobald ein Signal von einem Bremssensor empfangen wird.

7. Pedalfahrzeug (10) nach Anspruch 1 bis 6, **dadurch gekennzeichnet, dass** die elektrischen Stimulationen von einem Signal abhängen, das von einem Sensor geliefert wird, der die Neigung des Fahrzeugs misst.

8. Pedalfahrzeug (10) nach Anspruch 1 bis 7, **dadurch gekennzeichnet, dass** das Computerprogramm außerdem so konfiguriert ist, dass es die Stimulation der unteren Gliedmaßen verringert, wenn eine Verringerung der von den unteren Gliedmaßen erbrachten willentlichen Leistung festgestellt wird, z. B. bei Müdigkeit.

9. Pedalfahrzeug (10) nach einem der vorhergehenden Ansprüche, **dadurch gekennzeichnet, dass** das Datenmessmodul (60) einen Kraft- oder Drehmomentsensor (64a, 64b) umfasst, der an mindestens einem der ersten und zweiten Antriebselemente (18, 24) so angeordnet ist, dass er ein Signal an das Steuermodul (52) überträgt, das von der Kraft abhängt, die von mindestens einem der Gliedmaßen des Nutzers auf das mindestens eine der ersten und zweiten Antriebselemente (18, 24) ausgeübt wird.

10. Pedalfahrzeug (10) nach dem vorhergehenden Anspruch, **dadurch gekennzeichnet, dass** das Datenmessmodul (60) einen Drehgeschwindigkeitssensor umfasst, der an mindestens einem der ersten und zweiten Antriebselemente (18, 24) so angeordnet ist, dass er ein Signal an das Steuermodul (52) überträgt, das proportional zu der Kraft ist, die von mindestens einem der Gliedmaßen des Nutzers auf das mindestens eine der ersten und zweiten Antriebselemente (18, 24) ausgeübt wird.

11. Pedalfahrzeug (10) nach einem der vorhergehenden Ansprüche, **dadurch gekennzeichnet, dass** das Datenmessmodul (60) einen Winkelsensor (62) umfasst, der an mindestens einem der ersten und zweiten Antriebselemente (18, 21) angeordnet ist, 24) so angeordnet ist, dass er ein Signal an das Steuermodul (52) in Bezug auf die Winkelposition von Kurbeln (21, 27) des ersten oder zweiten Antriebselements (18, 24) überträgt, um die elektrischen Stimulationen in Abhängigkeit von der Winkelposition eines der ersten und zweiten Antriebselemente zu synchronisieren.

12. Pedalfahrzeug (10) nach einem der vorhergehenden Ansprüche, **dadurch gekennzeichnet, dass** das Steuermodul (52) eine erste Verarbeitungseinheit (54) zur Verarbeitung von Daten bezüglich der Absicht des Nutzers, eine zweite Verarbeitungseinheit (56) zur Verarbeitung von Daten bezüglich des mindestens einen zur Stimulation vorgesehenen Glieds und eine Steuereinheit (58) umfasst, die so konfiguriert ist, dass sie an die mindestens eine Elektrode (80) elektrische Stimulationen in Abhängigkeit von den durch die beiden Verarbeitungseinheiten (54, 56) übertragenen Daten überträgt.

13. Pedalfahrzeug (10) nach dem vorhergehenden Anspruch, **dadurch gekennzeichnet, dass** die Steuereinheit (58) so programmierbar ist, dass sie ein Trainingsprogramm eingeben kann, das einen Zielwert für die von den unteren Gliedmaßen ausgeübte Gesamtleistung enthält.

14. Pedalfahrzeug nach einem der vorhergehenden Ansprüche, **dadurch gekennzeichnet, dass** die von der stimulierten Gliedmaße ausgeübte Leistung so angezeigt werden kann, dass der Wert der resultierenden Leistung der elektrischen Stimulation von dem Wert der vom Nutzer absichtlich ausgeübten Leistung getrennt ist.

## Claims

1. **A pedal vehicle** (10) for moving along a route at the whim of a user, comprising a frame (12), a seat (14) supported by the frame (12), at least two wheels (15a, 15b, 15c) arranged respectively at the front and rear of the seat (14), a steering system (15), a first drive member (18) adapted to be operated by the user's upper limbs, a second drive member (24) adapted to be operated by the user's lower limbs, and a third drive member (25) adapted to be operated by the user's lower limbs, at least one brake (44) and an electrical stimulation device (50) comprising a control module (52), a data measurement module (60) and at least one electrode (80) adapted to stimulate at least one lower limb to be stimulated, the control module (52) being configured to process data relating to the user's intention and comprising a computer program configured to transmit to the at least one electrode (80) by the control module (52) electrical stimulations according to the data relating to the user's intention,
**characterised in that** said user intention data comprises the power or force intentionally developed by the user's upper limbs,
**in that** a step of determining the power intentionally developed by the lower limbs is performed,
and **in that** said electrical stimulations are determined so as to ensure a predetermined relationship between the power developed by the upper limbs and the total power developed by the lower limbs, said total power corresponding to the sum of the power developed intentionally by the lower limbs and the power developed by the lower limbs as a result of the stimulation.

2. Pedal vehicle (1O) according to claim 1, **characterized in that** said electrical stimulations (s) are determined as a function of the power respectively of the force intentionally developed by the user's upper limbs and of the power respectively of the force intentionally developed by the user's lower limbs.

3. A pedal vehicle (1O) according to claim 1 or 2, **characterised in that** said electrical stimulations are determined so as to ensure a given proportion between the power intentionally developed by the user's upper limbs and the total power developed by the lower limbs.

4. Pedal vehicle (1O) according to claim 1 to 3, **characterized in that** said electrical stimulations are stopped or reduced when the total power developed by a lower limb exceeds a predetermined value.

5. Pedal vehicle (1O) according to claim 1 to 4, **characterized in that** said electrical stimulations are stopped or reduced as soon as the measurement of the rotational speed of one of the first and second drive members exceeds a predetermined rotational speed.

6. Pedal vehicle (1O) according to claim 1 to 5, **characterized in that** said electrical stimulations are stopped upon receipt of a signal from a brake sensor.

7. Pedal vehicle (1O) according to claim 1 to 6, **characterized in that** said electrical stimulations are dependent on a signal delivered by a sensor measuring the inclination of the vehicle.

8. A pedal vehicle (1O) according to claim 1 to 7, **characterized in that** the computer program is further configured to reduce the stimulation of the lower limbs in case of detection of a reduction of the voluntary power provided by the lower limbs, for example in case of fatigue.

9. A pedal vehicle (10) according to any of the preceding claims, **characterized in that** the data measurement module (60) comprises a force or torque sensor (64a, 64b) arranged on at least one of the first and second drive members (18, 24) so as to transmit a signal to the control module (52) which is dependent on the force exerted on the at least one of the first and second drive members (18, 24) by at least one of the user's limbs.

10. A pedal vehicle (1O) according to the preceding claim, **characterized in that** the data measurement module (60) comprises a rotational speed sensor arranged on at least one of the first and second drive members (18, 24) so as to transmit a signal to the control module (52) proportional to the power exerted on the at least one of the first and second drive members (18, 24) by at least one of the user's limbs.

11. A pedal vehicle (10) according to any of the preceding claims, **characterized in that** the data measuring module (60) comprises an angular sensor (62) arranged on at least one of the first and second drive members (18, 24) so as to transmit a signal to the control module (52) relative to the angular position of cranks (21, 27) of the first or second drive member (18, 24) so as to synchronise the electrical stimulation as a function of the angular position of one of the first and second drive members.

12. A pedal vehicle (10) according to any of the preceding claims, **characterized in that** the control module (52) comprises a first processing unit (54) for processing data relating to the user's intention, a second processing unit (56) for processing data relating to said at least one limb to be stimulated and a management unit (58) configured to transmit to said at least one electrode (80) electrical stimulations as a function of the data transmitted by the two processing units (54, 56).

13. A pedal vehicle (10) according to the preceding claim, **characterized in that** the control unit (58) is programmable to enter a training program with a target value of the total power exerted by the lower limbs.

14. A pedal vehicle according to any of the preceding claims, **characterized in that** the power exerted by the stimulated limb can be displayed so that the value of the power resulting from the electrical stimulation is dissociated from the value of the power intentionally exerted by the user.
